# EUROPÄISCHE PATENTSCHRIFT

(11) **EP 2 955 537 B1**
(45) Veröffentlichungstag und Bekanntmachung des Hinweises auf die Patenterteilung: **07.08.2019**
(21) Anmeldenummer: 15171039.9
(22) Anmeldetag: 08.06.2015
(51) Int. Cl.: G01S 1/70

(54) **VORRICHTUNG UND VERFAHREN ZUR PRÄSENZERKENNUNG VON EINEM MOBILEN GERÄT**
DEVICE AND METHOD FOR DETECTING THE PRESENCE OF A MOBILE DEVICE
DISPOSITIF ET PROCÉDÉ DE RECONNAISSANCE DE PRÉSENCE D'UN APPAREIL MOBILE

(30) Priorität: 13.06.2014 DE 102014108364
(43) Veröffentlichungstag der Anmeldung: 16.12.2015
(73) Patentinhaber: Karl Storz SE & Co. KG, 78532 Tuttlingen (DE)
(72) Erfinder: Brüderle, Klaus, 78345 Moos (DE)
(74) Vertreter: Witte, Weller & Partner Patentanwälte mbB

(56) Entgegenhaltungen:
- DE-A1-102007 059 311
- US-A1- 2009 080 348
- US-A1- 2011 063 429
- US-A1- 2014 153 747

## Beschreibung

Die Erfindung betrifft eine Vorrichtung und ein Verfahren zur Präsenzerkennung von einem mobilen Gerät in einem Raum, insbesondere in einem medizinischen Behandlungsraum.

Die Ausgangssituation für die vorliegende Erfindung ist wie folgt. In einem Raum befinden sich zumindest ein mobiles Gerät und zumindest ein Zielgerät, das über das mobile Gerät gesteuert werden soll. Das mobile Gerät kann während eines üblichen Arbeitsablaufs aus dem Raum entfernt werden. Insbesondere besteht die Möglichkeit, das mobile Gerät auf einfache Weise in einen anderen Raum zu überführen und in dem anderen Raum einzusetzen.

Das Zielgerät hingegen soll als Einrichtung des Raums verstanden werden. Es ist bei dem Zielgerät daher nicht vorgesehen, das Zielgerät während eines normalen Arbeitsablaufs aus dem Raum zu entfernen. Zu den Zielgeräten zählen daher insbesondere solche Geräte, die für einen dauerhaften Verbleib in dem Raum ausgebildet sind.

Um das Zielgerät über das mobile Gerät steuern zu können, ist eine Gerätesteuereinheit zum Ansteuern des Zielgeräts vorhanden. Das mobile Gerät sendet Steuerbefehle, insbesondere mittels drahtloser Übertragung, an die Gerätesteuereinheit, die dann wiederum das Zielgerät ansteuert. Dabei vermittelt die Gerätesteuereinheit üblicherweise zwischen mobilem Gerät und Zielgerät und verifiziert die Steuerbefehle.

Diesen Aufbau findet man beispielsweise in einem Operationssaal, wo mittels einer Fernbedienung als mobiles Gerät ein Operationstisch als Zielgerät über eine drahtlose Verbindung ferngesteuert werden kann. Dabei ist es wichtig, dass der räumliche Bezug zwischen dem mobilen Gerät und dem Zielgerät gesichert ist, das heißt, das mobile Gerät und das Zielgerät müssen sich im selben Raum befinden, um eine sichere Bedienung zu gewährleisten. Dabei ist es häufig ein Kriterium für die sichere Bedienung, dass Bediener Auswirkungen seiner Eingaben im Prozessverhalten direkt erkennen kann.

Aufgrund der üblicherweise drahtlosen Verbindung zwischen dem mobilen Gerät und der Gerätesteuereinheit ist anders als bei kabelgebundenen Verbindungen nicht mehr sichergestellt, dass sich das mobile Gerät im selben Raum befindet wie das Zielgerät, nur weil eine Verbindung zwischen dem mobilen Gerät und dem Zielgerät besteht. Zudem ist eine eineindeutige Zuordnung in der Regel nicht gewünscht, da das mobile Gerät auch in anderen Räumen einsetzbar sein soll, allerdings eben nicht unbeabsichtigt.

Aufgrund der heute verfügbaren drahtlosen Übertragungstechniken ist es ohne weiteres möglich, dass sich das mobile Gerät in einem anderen Raum befindet, möglicherweise sogar weit entfernt von dem Raum befindet, in dem das Zielgerät steht. Es kann daher die Situation entstehen, dass ein Benutzer außerhalb des Raums versehentlich das Zielgerät bedient. Insbesondere kann die Situation entstehen, dass sich der Benutzer mit dem mobilen Gerät in einem ersten Raum befindet, um ein dort befindliches Zielgerät zu steuern, der Benutzer aber tatsächlich, also fälschlicher Weise, ein Zielgerät in einem zweiten Raum steuert. Solche Fehler sind gerade in medizinischen Behandlungsräumen wie z.B. einem Operationssaal besonders gefährlich.

Der Stand der Technik hat zu dieser Problematik bereits interessante Lösungsvorschläge gemacht. So wird beispielsweise in US 2011/0063429 beschrieben, dass man innerhalb eines Raums ein Ultraschallsignal aussendet und versucht, das Ultraschallsignal mit einem Steuermikrofon aufzufangen, das der Steuerung des Zielgeräts dient. Kann das Ultraschallsignal von dem Steuermikrofon erfasst werden, so erlaubt dies den Rückschluss, dass sich das Steuermikrofon im richtigen Raum befindet, das heißt, in dem Raum, in dem das Ultraschallsignal ausgesendet wird.

Eine weitere Idee ist es, ein mobiles Mikrofon für eine Sprachsteuerung mit einem RFID-Chip auszustatten. Erhält man bei einer Abfrage aller RFID-Chips in dem Raum auch die Identifikationsnummer des Mikrofons zurück, so kann man daraus schließen, dass sich das Mikrofon im richtigen Raum befindet, das heißt, in dem Raum, in dem die RFID-Abfrage durchgeführt wurde.

Trotz der großen Zuverlässigkeit der Verfahren aus dem Stand der Technik können doch bestimmte potenzielle Fehlersituationen möglicherweise nicht zuverlässig erfasst werden. Daher ist es eine Aufgabe der vorliegenden Erfindung, eine verbesserte Vorrichtung und ein verbessertes Verfahren zur Präsenzerkennung von einem mobilen Gerät in einem Raum aufzuzeigen. Dabei soll insbesondere auch in außergewöhnlichen und seltenen Konstellationen sichergestellt sein, dass ein mobiles Gerät wirklich nur dann als in einem Raum präsent erkannt wird, wenn das mobile Gerät auch tatsächlich in diesem Raum präsent ist.

US 2014/0153747 A1 zeigt eine Vorrichtung auf, die die Steuerung eines Geräts über ein Mikrofon absichert. Dafür wird in dem Raum, in dem sich das Mikrofon und das Gerät befindet, ein Überprüfungston ausgesendet. Kann der Überprüfungston auch in einem Signal detektiert werden, welches vom Mikrofon kommt, wird davon ausgegangen, dass sich das Mikrofon und das zu steuernde Gerät in demselben Raum befinden. Eine Ansteuerung des Geräts durch das Mikrofon ist dann möglich, andernfalls nicht.

US 2011/0063429 A1 offenbart ebenfalls eine Vorrichtung, die die Steuerung eines Geräts durch ein Mikrofon absichert. Hier wird überprüft, ob sich das Mikrofon in der Nähe des Geräts befindet. Wenn dies der Fall ist, kann das Gerät über das Mikrofon gesteuert werden, andernfalls nicht.

Gemäß einem ersten Aspekt der Erfindung wird die Aufgabe gelöst durch eine Vorrichtung gemäß Anspruch 1.

Eine Besonderheit der vorliegenden Erfindung liegt darin, dass mittels einer Regelschleife überprüft wird, ob sich das mobile Gerät in dem Raum befindet. Dabei ist das mobile Gerät mit seinem Signalempfänger Teil der Regelschleife. Der Signalempfänger ist derart in räumlicher Nähe zu dem mobilen Gerät angeordnet, dass sie sich im normalen Arbeitseinsatz gemeinsam in dem Raum befinden.

Die genannte Signalquelle ist vergleichbar mit dem Zielgerät für eine dauerhafte Anordnung in dem Raum ausgebildet. Das heißt, während des normalen Arbeitseinsatzes verbleibt die Signalquelle in dem Raum. Insgesamt sind damit das mobile Gerät und der Signalempfänger mobil und sind das Zielgerät und die Signalquelle stationär im Raum. Bei einer bevorzugten Ausgestaltung befindet sich auch die Gerätesteuereinheit stationär im Raum, dabei insbesondere als eine Einrichtung, die von Zielgerät und mobilem Gerät getrennt ist, oder als Teil des Zielgeräts.

Die Regelschleife ist derart ausgebildet, dass auf ein vorgebbares Sollwertsignal eingeregelt werden kann. Bei der Vorgabe des Sollwertsignals wird bevorzugt sichergestellt, dass das Sollwertsignal über einem Grundrauschen liegt, das im Raum vorhanden ist. Dadurch kann besonders gut sichergestellt werden, dass eine Unterscheidung zwischen dem Pilotsignal bzw. dem daraus resultierenden Raumsignal und dem im Raum vorhandenen Grundrauschen differenziert werden kann. Wenn die Amplitude des Sollwertsignals deutlich oberhalb der Amplitude des Grundrauschens im Raum liegt, kann bei einer bevorzugten Ausgestaltung über den Signalempfänger das Grundrauschen ermittelt werden und von dem Messsignal abgezogen werden. So kann ein ausreichend hoher Signal-Rauschabstand erzielt werden.

Für das Ansteuern der im Raum befindlichen Signalquelle ist die Signalsteuereinheit verantwortlich. Zu Beginn der Regelschleife steuert die Signalsteuereinheit die Signalquelle so an, dass die Signalquelle ein initiales Pilotsignal aussendet, insbesondere in den Raum aussendet. Da in der Praxis keine Informationen vorhanden sind, wie das Pilotsignal den Signalempfänger erreicht, sei es auf direktem Wege, durch Einfach- oder Mehrfachreflektionen oder Reflektionen über verschiedene Oberflächen, wird im Rahmen der Erläuterungen allgemein von einem Raumsignal gesprochen, das im Wesentlichen von dem Pilotsignal hervorgerufen wird und den Signalempfänger erreicht. Ein Grundrauschen im Raum wirkt sich eher nur minimal aus. Das Raumsignal kann an jeder Stelle des Raums unterschiedlich sein und kann in Abhängigkeit von der Ausrichtung des Signalempfängers selbst an der gleichen Stelle unterschiedlich stark erfasst werden.

Der Signalempfänger empfängt das Raumsignal und gibt ein Messsignal in Reaktion auf das empfangene Raumsignal aus. Dieses Messsignal wird an die Signalsteuereinheit zurückgekoppelt. Die Signalsteuereinheit gewinnt aus dem Messsignal ein Istwertsignal. Beinhaltet das Messsignal genau die Information, die mit dem vorgebbaren Sollwertsignal verglichen werden soll, so kann im einfachsten Fall das Istwertsignal dem Messsignal entsprechen. Enthält das Messsignal Informationen, die für einen Vergleich mit dem Sollwertsignal nicht erwünscht sind oder nicht benötigt werden, so kann das Istwertsignal als Teil des Messsignals aus dem Messsignal gewonnen werden. Hierzu kann das Messsignal insbesondere gefiltert werden, um im Ergebnis das Istwertsignal zu erhalten. Der Istwert bzw. das Istwertsignal wird dem Sollwert bzw. dem Sollwertsignal nachgeregelt.

Wenn das Istwertsignal gewonnen ist, steuert die Signalsteuereinheit die Signalquelle so an, dass sich das Istwertsignal dem Sollwertsignal mit dem nächsten Durchlauf der Regelschleife annähert, vorausgesetzt, dass das mobile Gerät tatsächlich präsent ist.

Anhand einer bevorzugten Ausführungsform soll eine mögliche Regelschleife näher erläutert werden. Hierfür sei angenommen, dass es das Ziel der Regelschleife ist, das Istwertsignal hinsichtlich seiner Amplitude auf eine mittels des Sollwertsignals vorgegebene Amplitude einzuregeln. Stellt die Signalsteuereinheit anhand des aus dem Messsignal gewonnenen Istwertsignals fest, dass die Amplitude des Istwertsignals zu gering ist, so steuert die Signalsteuereinheit die Signalquelle so an, dass das Pilotsignal in seiner Amplitude erhöht wird. Im umgekehrten Fall, wenn die Amplitude des Istwertsignals über der Amplitude des Sollwertsignals liegt, wird die Amplitude des Pilotsignals reduziert. Bei der Amplitude kann es sich dabei sowohl um eine Momentanamplitude handeln oder auch um eine effektive Amplitude, die sich im zeitlichen Mittel bei aus einer gepulsten Momentanamplitude ergibt. Dabei ist es bevorzugt, wenn die Feedbackschleife (Sprungantwortzeit) sehr viel kleiner ist als die Zeitspannen der Signalschwankungen, welche durch die Positionsänderungen des mobilen Gerätes (Bewegung durch den Bediener) hervorgerufen werden.

In gleicher Weise können auch andere Parameter eines Signals nachgeregelt werden. So werden bei anderen bevorzugten Ausführungsformen die Frequenz und/oder die Phase nachgeregelt. Stellt sich bei einem oder mehreren Durchläufen der Regelschleife eine Annäherung des Istwertsignals an das vorgegebene Sollwertsignal ein, so wird dies als Indiz dafür angesehen, dass sich das mobile Gerät in demselben Raum befindet wie die stationäre Signalquelle. Dies bedeutet wiederum, dass sich das mobile Gerät und das stationäre Zielgerät im selben Raum befinden. Eine Ansteuerung des Zielgeräts durch das mobile Gerät kann daher erlaubt werden. Bei einigen bevorzugten Ausführungsformen werden zusätzliche Plausibilitätsprüfungen vorgenommen, bevor ein Steuerbefehl vom mobilen Gerät an das Zielgerät zur Ausführung übertragen wird.

Stellt sich trotz der beschriebenen Regelung keine Annäherung des Istwertsignals an das Sollwertsignal ein, so ist dies ein Indiz dafür, dass sich das mobile Gerät nicht in demselben Raum befindet wie die stationäre Signalquelle. In diesem Fall ist es bei bevorzugten Ausgestaltungen vorgesehen, dass die Gerätesteuereinheit das Zielgerät nicht ansteuert, selbst wenn ein entsprechender Steuerbefehl vom mobilen Gerät empfangen wird.

Drei bevorzugte Varianten, um eine Ansteuerung des Zielgeräts durch das mobile Gerät in Abhängigkeit davon vorzunehmen, ob die Annäherung stattgefunden hat, sind wie folgt: a) Eine Ansteuerung des Zielgeräts durch das mobile Gerät wird unterbunden, wenn eine Annäherung des Istwertsignals an das Sollwertsignal nicht festgestellt werden kann. b) Eine Ansteuerung des Zielgeräts durch das mobile Gerät wird grundsätzlich erlaubt, sofern nicht das Fehlen einer Annäherung des Istwertsignals an das Sollwertsignal festgestellt wird. c) Eine Kombination der Varianten a) und b).

Bei einer bevorzugten Ausführungsform ist die Signalquelle eine von der Gerätesteuereinheit, dem mobilen Gerät und dem Zielgerät getrennte Vorrichtung. Bei einigen bevorzugten Ausgestaltungen ist die Signalsteuereinheit ein Teil der Gerätesteuereinheit oder ein Teil des Zielgeräts.

Die Signalquelle ist dafür ausgebildet, unterschiedliche Pilotsignale in Abhängigkeit von der Ansteuerung durch die Signalsteuereinheit auszusenden. Bei bevorzugten Ausgestaltungen ist die Signalquelle dafür ausgebildet, das Pilotsignal mittels Amplitudenmodulation und/oder Pulsweitenmodulation zu verändern. Bei bevorzugten Ausgestaltungen handelt es sich bei dem Istwertsignal um einen numerischen Istwert und bei dem Sollwertsignal um einen numerischen Sollwert.

Das mobile Gerät ist bevorzugt über eine drahtlose Verbindung mit der Gerätesteuereinheit oder, bei einigen vorteilhaften Ausgestaltungen, mit der Signalsteuereinheit verbunden. Hier kann insbesondere eine Verbindung über WLAN oder über Bluetooth eingesetzt werden.

Die Signalquelle ist dafür ausgebildet, ein optisches Signal auszusenden, und ist der Signalempfänger dafür ausgebildet, ein optisches Signal zu empfangen. Dies ist vorteilhaft, da das Pilotsignal nicht die Raumakustik beeinflusst und auch keine Notwendigkeit besteht, die Amplitude des Pilotsignals oberhalb eines normalen Geräuschpegels in dem Raum zu wählen. Dabei wird die Frequenz bzw. das Spektrum des optischen Signals insbesondere derart gewählt, dass das menschliche visuelle Empfinden in dem Raum nur minimal, bevorzugt gar nicht, beeinflusst wird.

Damit ist die Aufgabe vollständig gelöst.

Bei einer bevorzugten Ausgestaltung der Erfindung ist das optische Signal ein Infrarotsignal.

Es wird als vorteilhaft angesehen, wenn es sich bei dem optischen Signal um ein Infrarotsignal handelt. Unter einem Infrarotsignal soll insbesondere ein Signal mit einer Wellenlänge zwischen 750 und 3000 nm, bevorzugt zwischen 760 und 1400 nm (Nahinfrarotbereich) und besonders bevorzugt zwischen 770 und 1200 nm verstanden werden. Dabei wird insbesondere ein Bereich von 800 bis 1000 nm, bevorzugt 850 bis 985 nm und besonders bevorzugt 900 bis 975 nm als vorteilhaft angesehen. Konkret wird bei einer bevorzugten Ausgestaltung eine Signalquelle mit einer Wellenlänge von zumindest ungefähr 950 nm verwendet.

Da sich ein Infrarotsignal gut in einem Raum verteilt und von einem Benutzer nicht oder zumindest nicht als störend wahrgenommen wird, wird diese Ausgestaltung als vorteilhaft angesehen. Die Signalquelle ist hier dafür ausgebildet, das Pilotsignal als optisches Signal auszusenden. Das Istwertsignal ist insbesondere ein Infrarotanteil des detektierten Raumsignals. Wenn das Raumsignal den Signalempfänger bereits auf den Infrarotanteil gefiltert erreicht, kann bei einer bevorzugten Ausführungsform das Messsignal direkt als Istwertsignal verwendet werden. Bei entsprechendem Messsignal kann natürlich auch bei allen anderen Ausgestaltungen das Messsignal direkt als Istwertsignal verwendet werden. Außerdem wurde im Rahmen der Erfindung erkannt, dass sich optische Signale einfacher durch bauliche Maßnahmen begrenzen lassen als Funksignale.

Bei einer weiteren vorteilhaften Ausgestaltung ist das Istwertsignal ein Infrarotanteil des detektierten Raumsignals.

Diese Ausgestaltung ist vorteilhaft, da mittels des Signalempfängers ein größerer Wellenlängenbereich empfangen werden kann, aber lediglich ein Infrarotanteil des detektierten Raumsignals in das Istwertsignal überführt wird, das dann wiederum mit dem Sollwertsignal verglichen wird.

Bei einer bevorzugten Ausgestaltung ist das Istwertsignal ein Istwert der Luminanz oder ein Istwert der Helligkeit oder ein Istwert der Intensität des detektierten Raumsignals.

Diese Ausgestaltung ist messtechnisch gut zu realisieren.

Bei einer weiteren Ausgestaltung ist die Gerätesteuereinheit dafür ausgebildet, zu ermitteln, ob die Annäherung innerhalb eines Toleranzfensters erfolgt, wobei das Toleranzfenster durch eine vorgebbare Höchstdauer für die Annäherung und/oder einen vorgebbaren Höchstabstand von Istwertsignal und Sollwertsignal definiert ist.

Diese Ausgestaltung ermöglicht es auf effektive Weise, festzustellen, ob eine Annäherung stattgefunden hat, eine Ansteuerung des Zielgeräts also grundsätzlich ermöglicht werden soll, oder ob eine Annäherung nicht stattgefunden hat. Wenn sich das mobile Gerät in demselben Raum befindet, in dem die Regelschleife über die Signalquelle realisiert ist, wird eine Annäherung des Istwertsignals an das Sollwertsignal erwartet. In Abhängigkeit davon, wie die Regelschleife ausgebildet ist, kann man entweder anhand theoretischer Überlegungen oder empirischer Messungen ermitteln, wie schnell die Annäherung stattfindet und/oder wie gering der Abstand von Istwertsignal und Sollwertsignal wird.

Wenn man beispielsweise feststellt, dass eine Annäherung üblicherweise innerhalb von 0,5 s stattfindet, kann man das Toleranzfenster z.B. doppelt so groß, also 1 s, festlegen. Findet eine Annäherung in diesem Zeitbereich des Toleranzfensters statt, ist dies ein Indiz dafür, dass sich das mobile Gerät in demselben Raum befindet. In gleicher Weise kann man überprüfen, wie nah das Istwertsignal dem Sollwertsignal kommen kann. Wird ein vorgegebener Höchstabstand zwischen Istwertsignal und Sollwertsignal unterschritten, so ist dies ein Indiz dafür, dass sich das mobile Gerät in demselben Raum befindet.

Bei bevorzugten Ausgestaltungen wird das Toleranzfenster sowohl im Hinblick auf die Zeit als auch im Hinblick auf den Höchstabstand definiert. Dies bedeutet, nur wenn die Annäherung innerhalb einer bestimmten Zeit stattfindet und dabei einen bestimmten Höchstabstand unterschreitet, wird auf die Präsenz des mobilen Geräts in demselben Raum geschlossen. Dabei kann das Toleranzfenster auch dynamisch gesteuert werden, so dass insbesondere auch bei einer langsamen Annäherung, die aber zu einem besonders geringen Abstand zwischen Istwertsignal und Sollwertsignal führt oder bei einem eigentlich zu großen Abstand, der aber besonders schnell erreicht wurde, die Präsenz des mobilen Geräts in demselben Raum angenommen wird.

Bei einer weiteren vorteilhaften Ausgestaltung ist die Vorrichtung dafür ausgebildet, das Sollwertsignal über der Zeit zu ändern.

Diese Ausgestaltung erhöht die Sicherheit bei der Präsenzerkennung des mobilen Geräts. Hierzu wird in zeitlichen Abständen das Sollwertsignal geändert. Dies bedeutet für die Regelschleife, dass das Istwertsignal nun einem neuen Sollwertsignal nachgeführt werden muss. Kann das Istwertsignal nach einer Änderung des Sollwertsignals nicht mehr dem Sollwertsignal angenähert werden, so zeigt dies an, dass sich das mobile Gerät nicht oder nicht mehr in dem Raum befindet. Bei einer bevorzugten Ausgestaltung wird für die Annäherung des Istwertsignals an das geänderte Sollwertsignal ein Toleranzfenster definiert, innerhalb dessen die Annäherung erfolgen muss. Für die Ausgestaltungsmöglichkeiten des Toleranzfensters wird auf das weiter oben beschriebene Toleranzfenster verwiesen. Das Prinzip der Rückkopplung und der erwarteten Annäherung wird also beibehalten, hat aber noch zusätzlich einen Signalsprung, der zeitlich verifiziert werden kann.

Der Signalsprung erzeugt bevorzugt eine deutliche Änderung. Unter einer deutlichen Änderung soll ein Anstieg von mindestens 10 %, bevorzugt mindestens 25 %, besonders bevorzugt von mindestens 100 % und insbesondere von mindestens 250 % verstanden werden. Ebenso soll eine Reduzierung von 100 % auf höchstens 90 %, bevorzugt auf höchstens 80 %, besonders bevorzugt auf höchstens 50 % und insbesondere auf höchstens 40 % als deutliche Änderung verstanden werden. Bei anderen bevorzugten Ausführungsformen unter einer deutlichen Änderung soll ein Anstieg um einen Faktor von mindestens 5, bevorzugt mindestens 10, besonders bevorzugt von mindestens 25 und insbesondere von mindestens 100 verstanden werden. Ebenso soll eine Reduzierung um einen Faktor von höchstens 0,2, bevorzugt höchstens 0,1, besonders bevorzugt höchstens 0,04 und insbesondere höchstens 0,01 als deutliche Änderung verstanden werden. Es wird insbesondere ein Bereich von 10 bis 100 bzw. 0,1 bis 0,01 als vorteilhaft angesehen.

Bei einer weiteren vorteilhaften Ausgestaltung repräsentiert das Messsignal eine zeitliche Abfolge des vom Signalempfänger empfangenen Raumsignals.

Diese Ausgestaltung bietet erweiterte Möglichkeiten bei der Auswertung des Messsignals, insbesondere bei der Gewinnung des Istwertsignals aus dem Messsignal. Sollen beispielsweise zeitliche Veränderungen des Messsignals berücksichtigt werden, so kann dies durch die Auswertung von Informationen zu unterschiedlichen Zeiten unterstützt werden. Insbesondere wenn das Messsignal Informationen zu einem Zeitpunkt enthält, zu dem die Signalquelle sendet, und zu einem zweiten Zeitpunkt, zu dem die Signalquelle nicht sendet, kann anhand einer Differenzanalyse, hier insbesondere die sog. "Two Samples Difference" insbesondere eine Trennung von konstanten Anteilen und dynamischen Anteilen im Messwertsignal vorgenommen werden. Bei einer bevorzugten Ausgestaltung enthält das Istwertsignal lediglich die dynamischen Anteile aus dem Messsignal, insbesondere solche Anteile, die sich innerhalb einer Sekunde ändern können, und enthält keine konstanten Anteile, insbesondere solche, die sich innerhalb einer Sekunde nicht oder lediglich geringfügig ändern.

Bei einer weiteren vorteilhaften Ausgestaltung der Erfindung ist der Signalempfänger eine Kamera und ist, insbesondere, das Messsignal eine zeitliche Abfolge von Bildern, die von der Kamera aufgenommen wurden.

Diese Ausgestaltung ermöglicht eine einfache Erfassung von optischen Signalen. Da bei der Kamera nicht eine hohe Bildqualität im Vordergrund stehen muss, sondern das Empfangen des durch das Pilotsignal hervorgerufenen Raumsignals, können bei bevorzugten Ausgestaltungen kostengünstige Kameras eingesetzt werden. Werden in dem Messsignal eine zeitliche Abfolge von mindestens zwei Bildern übertragen, so können, wie oben erläutert, auf einfache Weise dynamische und konstante Anteile im Messsignal identifiziert werden. Bei bevorzugten Ausgestaltungen ist das Pilotsignal gepulst oder in der Intensität variiert.

Bei einer alternativen bevorzugten Ausgestaltung werden die Bilddaten bereits vor ihrer Einkopplung in das Messsignal verarbeitet, insbesondere von dem mobilen Gerät, so dass das Messsignal nur noch die Analysedaten enthält. Auf diese Weise kann das Datenvolumen, das mit dem Messsignal übertragen wird, reduziert werden. Insbesondere wenn nur die Gesamthelligkeit oder die Gesamtluminanz oder die Gesamtintensität der Bilder ausgewertet werden soll, ist es vorteilhaft, wenn diese Werte ausgerechnet werden und dann lediglich diese Werte in das Messsignal eingekoppelt und an die Signalsteuereinheit übertragen werden.

Bei einer weiteren vorteilhaften Ausgestaltung ist die Signalquelle dafür ausgebildet, eine gewünschte Signalstärke durch Abgabe einer variablen Sendeleistung und/oder durch eine entsprechend gepulste konstante Sendeleistung bereitzustellen.

Diese Ausgestaltung ermöglicht es, der Regelschleife eine hinreichende Flexibilität für die Annäherung des Istwertsignals an das Sollwertsignal zu geben. Wenn die Signalquelle eine variable Sendeleistung hat, kann auf einfache Weise die Sendeleistung erhöht werden, wenn das Istwertsignal unterhalb des Sollwertsignals liegt und umgekehrt. Wenn die Signalquelle ein gepulstes Pilotsignal aussendet, kann die Pulslänge erhöht werden und/oder die Pausenzeit zwischen einzelnen Pulsen verringert werden, wenn das Istwertsignal unterhalb des Sollwertsignals liegt und umgekehrt. Bei einigen bevorzugten Ausgestaltungen kann die Signalquelle sowohl hinsichtlich ihrer Sendeleistung (Amplitudenmodulation) als auch im Hinblick auf die ausgesandten Pulse (Pulsweitenmodulation) verändert werden.

Bei einer weiteren vorteilhaften Ausgestaltung ist der Signalempfänger eine im mobilen Gerät eingebaute Kamera, der ein infrarotdurchlassendes Filter aufgesetzt ist und ist, insbesondere, zusätzlich eine Weitwinkellinse aufgesetzt.

Diese Ausgestaltung ist besonders kostengünstig zu realisieren. Als mobiles Gerät zur Steuerung von Zielgeräten kann somit ein handelsüblicher tragbarer Computer, insbesondere ein Tablet, verwendet werden. Derartige Geräte verfügen in aller Regel bereits ab Werk über eine eingebaute Kamera. Die Bildqualität dieser Kameras ist zwar beschränkt, doch wurde erkannt, dass auch eine eingebaute Kamera zum Zwecke der hier offenbarten Präsenzerkennung ausreichend ist.

Wenn es sich bei dem optischen Signal um ein Infrarotsignal handelt, wird mittels des infrarotdurchlassenden Filters sichergestellt, dass im Wesentlichen nur Infrarotanteile die Kamera erreichen. Dadurch erreicht das normale, sichtbare Umgebungslicht die Kamera nicht oder nur stark gedämpft. Dies erleichtert die Auswertung von Bildern der Kamera hinsichtlich der im Bild befindlichen Infrarotanteile.

Wenn bei einer bevorzugten Ausgestaltung zusätzlich eine Weitwinkellinse aufgesetzt ist, wird die Abhängigkeit des von der Kamera empfangenen Raumsignals von der Positionierung des mobilen Geräts im Raum verringert. Bei bevorzugten Ausgestaltungen werden hier eine Fischaugenlinse (fish eye lens), eine 360°-Linse, eine Kugellinse oder eine Panoramalinse verwendet. Auf diese Weise kann insbesondere einer unruhigen Charakteristik des Messsignals einer Tablet-Kamera Rechnung getragen werden.

Bei einer bevorzugten Ausgestaltung der Erfindung ist die räumliche Nähe von Signalempfänger und mobilem Gerät derart ausgestaltet, dass sich das mobile Gerät und der Signalempfänger in einem Abstand von weniger als 5 m, bevorzugt weniger als 2 m, besonders bevorzugt weniger als 1 m und insbesondere weniger als 0,5 m zueinander befinden. Wenn das mobile Gerät bei einer bevorzugten Ausgestaltung einen Gerätewagen aufweist, ist bei einer bevorzugten Ausgestaltung eine Kamera als Signalempfänger konstruktiv am Gerätewagen befestigt.

Mittels dieser Ausgestaltung kann sichergestellt werden, dass sich das mobile Gerät nicht in einem ersten Raum befinden kann, wo ein Zielgerät gesteuert werden kann, und sich der Signalempfänger in einem zweiten Raum befindet, wo ein anderes Zielgerät gesteuert werden kann. Bei einer bevorzugten Ausgestaltung ist die räumliche Nähe von mobilem Gerät und Signalempfänger konstruktiv so ausgestaltet, dass sie von einem Benutzer während des normalen Arbeitsablaufs nicht verändert werden kann oder zumindest nicht über einen Höchstabstand hinaus verändert werden kann. Bei einer anderen bevorzugten Ausgestaltung ist die räumliche Nähe alternativ oder zusätzlich konstruktiv so ausgestaltet, dass mobiles Gerät und Signalempfänger nicht voneinander getrennt werden können.

Bei einer weiteren bevorzugten Ausgestaltung ist die räumliche Nähe derart ausgestaltet, dass der Signalempfänger in das mobile Gerät eingebaut ist.

Auf diese Weise kann sichergestellt werden, dass der Signalempfänger nicht vom mobilen Gerät getrennt werden kann, zumindest nicht im normalen Arbeitsablauf und nicht ohne umfangreiche technische Eingriffe.

Bei einer weiteren Ausgestaltung weist die Vorrichtung ein zusätzliches mobiles Gerät mit einem zusätzlichen Signalempfänger zum Empfangen des Raumsignals auf, wobei der zusätzliche Signalempfänger dafür ausgebildet ist, ein zusätzliches Messsignal in Reaktion auf das empfangene Raumsignal auszugeben, wobei die Gerätesteuereinheit ferner dafür ausgebildet ist, eine Ansteuerung des Zielgeräts durch das zusätzliche mobile Gerät in Abhängigkeit davon vorzunehmen, ob eine Änderung des Sollwertsignals zu einer entsprechenden Änderung des zusätzlichen Messsignals führt.

Diese Ausgestaltung ermöglicht es, die Präsenz von weiteren mobilen Geräten, insbesondere in dem Raum, zu überprüfen. Während die Präsenz des mobilen Geräts nach einer Änderung des Sollwertsignals dadurch überprüft wird, ob es das mobile Gerät schafft, das Istwertsignal wieder dem Sollwertsignal anzunähern, wird für das zusätzliche mobile Gerät überprüft, ob die Änderung des Sollwertsignals, was zu einer Änderung des Pilotsignals und des Raumsignals führt, auch in dem zusätzlichen Messsignal festzustellen ist. Dabei ist es bevorzugt, wenn sich das Sollwertsignal deutlich ändert. Was unter einer deutlichen Änderung verstanden werden soll, wurde bereits zuvor erläutert.

Die deutliche Änderung des Sollwertsignals gegenüber dem Istwertsignal bewirkt eine entsprechende Änderung des Pilotsignals, da die Signalsteuereinheit versucht, auf das neue Sollwertsignal nachzuregeln. Wird das Sollwertsignal beispielsweise deutlich erhöht, so führt dies über die Regelschleife auch zu einer Erhöhung des Pilotsignals und damit des Raumsignals. Wenn diese Änderung in entsprechender Weise auch von dem zusätzlichen mobilen Gerät erfasst wird, ggf. mit einer geringen Zeitverzögerung, so ist dies ein Indiz dafür, dass sich das zusätzliche mobile Gerät in demselben Raum befindet. Ändert sich das zusätzliche Messsignal nicht oder in entgegengesetzter Richtung, so ist dies ein Indiz dafür, dass sich das zusätzliche mobile Gerät nicht in demselben Raum befindet.

Bei einer weiteren bevorzugten Ausgestaltung wird vor dem Signalempfänger, der bevorzugt eine im mobilen Gerät eingebaute Kamera ist, eine nachleuchtende Beschichtung, eine nachleuchtende Folie oder ein nachleuchtendes Element angeordnet.

Licht kann "flüchtig" sein, sprich, die Helligkeitssignatur im Raum kann sich sehr schnell ändern. Der Signalempfänger muss also schnell genug sein, um dem zu folgen. Es ist daher bevorzugt, wenn diese Dynamik, aus Sicht des Signalempfängers gedämpft oder geglättet wird. Dazu werden bei der weiteren bevorzugten Ausgestaltung nachleuchtende oder "lichtspeichernde" Beschichtungen, Folien oder Elemente verwendet. Dadurch können die Helligkeitsänderungen für den Signalempfänger gedämpft werden. Er könnte langsamer arbeiten, insbesondere weniger Bilder pro Sekunde aufnehmen, und könnte daher weniger leistungsfähig sein.

Gemäß einem weiteren Aspekt der Erfindung wird die Aufgabe gelöst durch ein Verfahren gemäß Anspruch 14.

Bei einer bevorzugten Ausgestaltung weist das Verfahren ferner die folgenden Schritte auf:
- Ausgeben eines zusätzlichen Messsignals basierend auf dem von einem zusätzlichen mobilen Gerät empfangenen Raumsignal,
- Ändern des Sollwertsignals,
- Ansteuern des Zielgeräts oder eines zusätzlichen Zielgeräts durch das zusätzliche mobile Gerät in Abhängigkeit davon, ob das Ändern des Sollwertsignals zu einer entsprechenden Änderung des zusätzlichen Messsignals führt.

Gemäß einem weiteren Aspekt wird die Aufgabe gelöst durch einen medizinischen Behandlungsraum, in dem eine zuvor beschriebene Vorrichtung zur Präsenzerkennung angeordnet ist und wobei sich zumindest die Gerätesteuereinheit, die Signalquelle, die Signalsteuereinheit und das Zielgerät in dem medizinischen Behandlungsraum befinden, insbesondere stationär befinden.

Dabei ist es bevorzugt, wenn zwei medizinische Behandlungsräume unterschiedliche Sollwertsignale haben und dass bei einer Änderung eines Sollwertsignals in einem Behandlungsraum die Änderung auf ein Sollwertsignals erfolgt, das von dem Sollwertsignal des anderen Behandlungsraums verschieden ist, bevorzugt deutlich verschieden ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung näher dargestellt und werden in der nachfolgenden Beschreibung näher erläutert. Es zeigen:
- Fig. 1: eine erste Ausführungsform einer Vorrichtung zur Präsenzerkennung;
- Fig. 2: eine zweite Ausführungsform einer Vorrichtung zur Präsenzerkennung;
- Fig. 3: eine dritte Ausführungsform einer Vorrichtung zur Präsenzerkennung;
- Fig. 4: eine vierte Ausführungsform einer Vorrichtung zur Präsenzerkennung;
- Fig. 5: eine erste Ausführungsform eines Verfahrens zur Präsenzerkennung;
- Fig. 6: eine zweite Ausführungsform eines Verfahrens zur Präsenzerkennung;
- Fig. 7: ein Ausführungsbeispiel eines zeitlichen Verlaufs, wenn eine Annäherung stattfindet;
- Fig. 8: ein Ausführungsbeispiel eines zeitlichen Verlaufs, wenn eine Annäherung nicht stattfindet;
- Fig. 9: ein erstes Bild aus einem beispielhaften Messsignal; und
- Fig. 10: ein zweites Bild aus dem beispielhaften Messsignal.

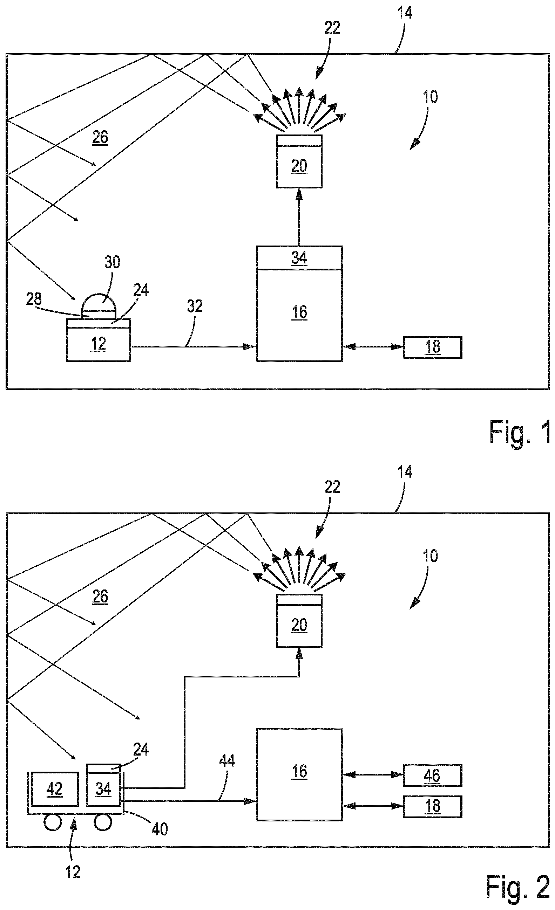

Fig. 1 zeigt eine Vorrichtung 10 zur Präsenzerkennung von einem mobilen Gerät 12 in einem Raum 14. Die Vorrichtung 10 weist eine Gerätesteuereinheit 16 zum Ansteuern eines Zielgeräts 18 auf. Das mobile Gerät 12 ist dafür ausgebildet, das Zielgerät 18 über die Gerätesteuereinheit 16 anzusteuern.

Die Vorrichtung 10 weist ferner eine Signalquelle 20 zum Aussenden eines Pilotsignals 22 auf, wobei das Pilotsignal 22 hier durch mehrere Pfeile symbolisch dargestellt ist. Die Vorrichtung weist ferner einen Signalempfänger 24 auf, der dafür ausgebildet ist, ein durch das Pilotsignal 22 hervorgerufenes Raumsignal 26 zu empfangen. Das Raumsignal 26 ist hier durch lange dünne Pfeile symbolisiert und erfüllt im Wesentlichen den gesamten Raum 14, wobei das Raumsignal 26 ortsvariant ist, das heißt, an unterschiedlichen Stellen des Raums 14 ist auch das Raumsignal 26 unterschiedlich.

Der Signalempfänger 24 ist in räumlicher Nähe zu dem mobilen Gerät 12 angeordnet, wobei der Signalempfänger 24 hier eine Kamera ist, die in dem mobilen Gerät 12 eingebaut ist. Dem Signalempfänger 24 ist hier ein infrarotdurchlassendes Filter 28 aufgesetzt. Dem Filter 28 ist zusätzlich eine Weitwinkellinse 30 aufgesetzt. Der Signalempfänger 24 ist dafür ausgebildet, ein Messsignal 32 in Reaktion auf das empfangene Raumsignal 26 auszugeben.

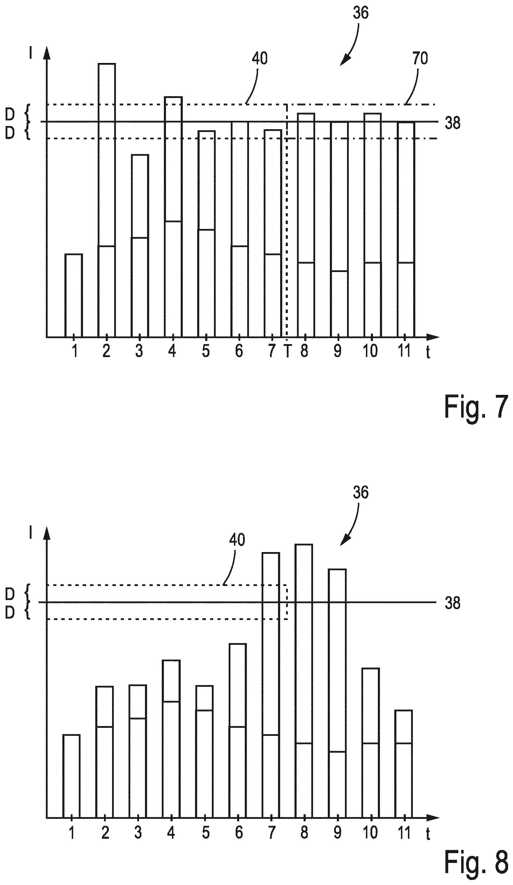

Die Vorrichtung 10 weist außerdem eine Signalsteuereinheit 34 auf, die dafür ausgebildet ist, die Signalquelle 20 anzusteuern. Die Signalsteuereinheit 34 ist außerdem dafür ausgebildet, die Signalquelle 20 in Abhängigkeit von einem aus dem Messsignal 32 gewonnenen Istwertsignal 36 (siehe Fig. 7) so anzusteuern, dass über der Zeit t (siehe Fig. 7) eine Annäherung des Istwertsignals 36 an ein vorgebbares Sollwertsignal 38 (siehe Fig. 7) stattfindet. Die Gerätesteuereinheit 16 ist dafür ausgebildet, eine Ansteuerung des Zielgeräts 18 durch das mobile Gerät 12 in Abhängigkeit davon vorzunehmen, ob die Annäherung stattgefunden hat.

Die Signalquelle 20 ist dafür ausgebildet, das Pilotsignal 22 als optisches Signal auszusenden. Der Signalempfänger 24 ist dafür ausgebildet, das Raumsignal 26 als ein optisches Signal zu empfangen. Dabei ist das optische Signal insbesondere ein Infrarotsignal. Das Istwertsignal 36 ist insbesondere ein Infrarotanteil des detektierten Raumsignals 26. Wenn das Raumsignal 26 den Signalempfänger 24 bereits auf den Infrarotanteil gefiltert erreicht, kann bei einer bevorzugten Ausführungsform das Messsignal 32 direkt als Istwertsignal 36 verwendet werden.

Das Istwertsignal 36 ist hier ein Istwert der Intensität des detektierten Raumsignals 26. Bei anderen bevorzugten Ausgestaltungen ist das Istwertsignal 36 ein Istwert der Luminanz oder ein Istwert der Helligkeit des detektierten Raumsignals 26. Die Gerätesteuereinheit 16 ist dafür ausgebildet, zu ermitteln, ob die Annäherung innerhalb eines Toleranzfensters 40 (siehe Fig. 7) erfolgt, wobei das Toleranzfenster 40 durch eine vorgebbare Höchstdauer T (siehe Fig. 7) für die Annäherung und/oder einen vorgebbaren Höchstabstand D (siehe Fig. 7) von Istwertsignal 36 und Sollwertsignal 38 definiert ist.

Die Vorrichtung 10 ist dafür ausgebildet, das Sollwertsignal 38 über der Zeit t zu ändern. Bei der hier gezeigten Ausführungsform ist insbesondere die Gerätesteuereinheit 16 dafür ausgebildet, das Sollwertsignal 38 über der Zeit t zu ändern. Das Messsignal 32 kann eine zeitliche Abfolge des vom Signalempfänger 24 empfangenen Raumsignals 26 repräsentieren. Im hier gezeigten Fall, bei dem der Signalempfänger 24 eine Kamera ist, ist das Messsignal 32 eine zeitliche Abfolge von Bildern, die von der Kamera aufgenommen wurden.

Die Signalquelle 20 ist dafür ausgebildet, eine gewünschte Signalstärke durch eine entsprechend gepulste konstante Sendeleistung bereitzustellen. In Abhängigkeit davon, wie lange der Puls der konstanten Sendeleistung ist und wie lange die Pause ist, während der die Signalquelle 20 nicht sendet, ergibt sich eine effektive Sendeleistung, die variiert werden kann. Bei anderen Ausführungsformen verfügt die Signalquelle 20 über eine variable Sendeleistung, wobei die Signalquelle 20 dann auch ohne Pause senden kann. Bei weiteren bevorzugten Ausgestaltungen werden beide Möglichkeiten der Variation der gewünschten Signalstärke kombiniert.

Fig. 2 zeigt eine zweite Ausführungsform einer Vorrichtung 10 zur Präsenzerkennung. Es gelten hier alle Ausführungen, die im Zusammenhang mit Fig. 1 gemacht worden sind. Zudem werden hier und im Nachfolgenden die bereits eingeführten Bezugszeichen weiter verwendet und zeigen gleiche oder funktional gleiche Elemente an. Im Unterschied zur Fig. 1 ist das mobile Gerät 12 hier eine Kombination von einem Wagen 40 und einem auf dem Wagen 40 positionierten Gerät 42. Der Signalempfänger 24 ist hier am Wagen 40 angeordnet, wobei aber durch die Befestigung des Geräts 42 sichergestellt ist, dass der Signalempfänger 24 nicht von dem mobilen Gerät 12 getrennt werden kann, also weder vom Wagen 40 noch vom Gerät 42. Außerdem ist die Signalsteuereinheit 34 hier zusammen mit dem Signalempfänger 24 ausgeführt. Da das Messsignal 32 bei dieser Ausgestaltung den Verbund von Signalempfänger 24 und Signalsteuereinheit 34 nicht verlässt, ist das Messsignal 32 hier nicht dargestellt.

Wenn die Regelschleife über die Signalquelle 20 zu einer Annäherung führt, wird ein Freigabesignal 44 gesendet, das es dem mobilen Gerät 12 ermöglicht, das Zielgerät 18 zu steuern. Schließlich ist bei der zweiten Ausführungsform noch ein zusätzliches Zielgerät 46 vorhanden, das gleichzeitig oder alternativ vom mobilen Gerät 12 aus gesteuert werden kann.

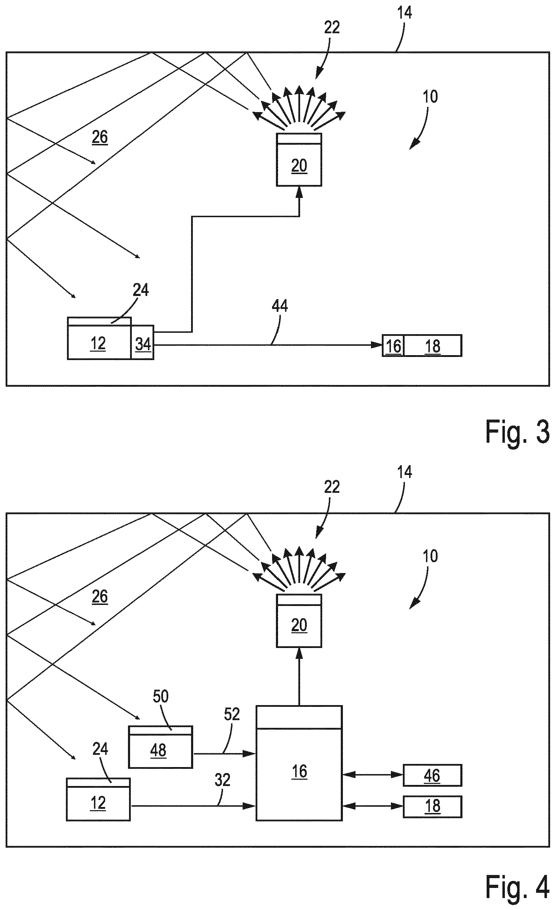

Fig. 3 zeigt eine dritte Ausführungsform einer Vorrichtung 10 zur Präsenzerkennung. Es gelten hier die Ausführungen zu den einzelnen Elementen, die im Zusammenhang mit Fig. 1 gemacht worden sind. Bei dieser Ausgestaltung ist die Gerätesteuereinheit 16 in dem Zielgerät 18 integriert. Wenn die Regelschleife über die Signalquelle 20 zu einer Annäherung führt, wird ein Freigabesignal 44 gesendet, das es dem mobilen Gerät 12 ermöglicht, das Zielgerät 18 zu steuern. Die Signalsteuereinheit 34 ist hier Bestandteil des mobilen Geräts 12.

Fig. 4 zeigt eine vierte Ausführungsform einer Vorrichtung 10 zur Präsenzerkennung. Es gelten hier die Ausführungen zu den einzelnen Elementen, die im Zusammenhang mit Fig. 1 und Fig. 2 gemacht worden sind. Die Vorrichtung 10 weist außerdem ein zusätzliches mobiles Gerät 48 mit einem zusätzlichen Signalempfänger 50 zum Empfangen des Raumsignals 26 auf. Der zusätzliche Signalempfänger 50 ist dafür ausgebildet, ein zusätzliches Messsignal 52 in Reaktion auf das empfangene Raumsignal 26 auszugeben. Die Gerätesteuereinheit 16 ist hier ferner dafür ausgebildet, eine Ansteuerung des Zielgeräts 18 oder des zusätzlichen Zielgeräts 46 durch das zusätzliche mobile Gerät 48 in Abhängigkeit davon vorzunehmen, ob eine Änderung des Sollwertsignals 38 zu einer entsprechenden Änderung des zusätzlichen Messsignals 52 führt. Auf diese Weise können auch beliebig viele weitere zusätzliche Geräte hinsichtlich ihrer Präsenz erfasst werden.

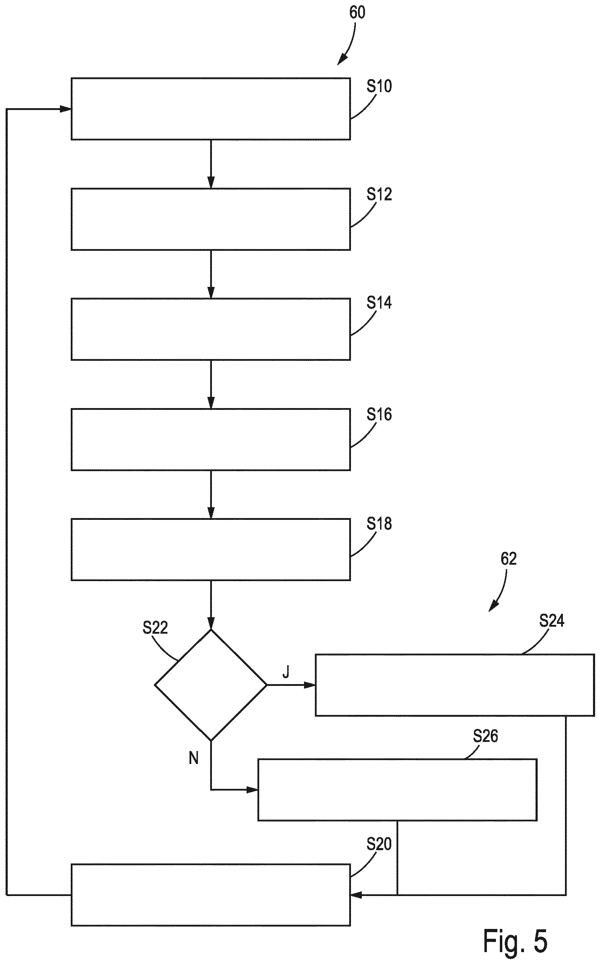

Fig. 5 zeigt ein erstes Ausführungsbeispiel eines Verfahrens 60 zur Präsenzerkennung von einem mobilen Gerät 12 in einem Raum 14. Das Verfahren 60 weist zunächst den Schritt S10 auf, bei dem eine Signalquelle 20 zum Aussenden eines Pilotsignals 22 angesteuert wird. Im Schritt S12 wird ein durch das Pilotsignal 22 hervorgerufenes Raumsignal 26 empfangen. Basierend auf dem von dem mobilen Gerät 12 empfangenen Raumsignal 26 wird im Schritt S14 ein Messsignal 32 ausgegeben. Im Schritt S16 wird aus dem Messsignal 26 ein Istwertsignal 36 gewonnen.

Ein Vergleich des Istwertsignals 36 mit einem vorgebbaren Sollwertsignal 38 findet im Schritt S18 statt. In einem Schritt S20 wird das Ansteuern der Signalquelle 20 angepasst, so dass über der Zeit eine Annäherung des Istwertsignals 36 an das vorgebbare Sollwertsignal 38 stattfindet.

In einem Block 62 wird das Zielgerät 18 durch das mobile Gerät 12 in Abhängigkeit davon angesteuert, ob die Annäherung stattgefunden hat. Dabei wird zunächst in einem Vergleichsschritt S22 ermittelt, ob die Annäherung stattgefunden hat, siehe hierzu auch nachfolgend die Erläuterung in Fig. 7. Ist dies der Fall, verzweigt das Verfahren über den Zweig J zum Schritt S24, in dem es dem mobilen Gerät 12, insbesondere durch ein Freigabesignal 44, ermöglicht wird, das Zielgerät 18 zu steuern. Hat keine Annäherung stattgefunden, so verzweigt das Verfahren über den Zweig N zum Schritt S26, wo es unterbunden wird, dass das mobile Gerät 12 das Zielgerät 16 ansteuert.

Nachdem die Ansteuerung der Signalquelle im Schritt S20 angepasst wurde, wird das Verfahren mit dem Schritt S10 fortgesetzt, das heißt, die Signalquelle 20 sendet ein angepasstes Pilotsignal 22.

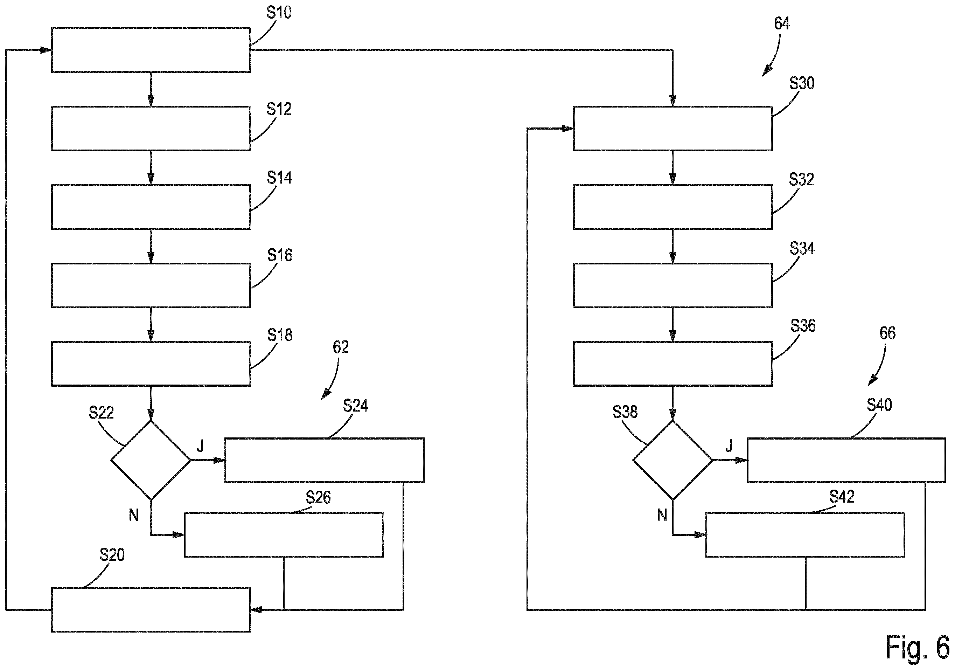

Fig. 6 zeigt eine zweite Ausführungsform eines Verfahrens 64 zur Präsenzerkennung. Der linke Teil der Darstellung entspricht der Darstellung aus Fig. 5. Alle im Kontext der Fig. 5 vorgenommenen Erläuterungen gelten daher auch hier.

Zusätzlich zur Fig. 5 wird hier im Schritt S30 das durch das Pilotsignal 22 hervorgerufene Raumsignal 26 nun auch von einem zusätzlichen Signalempfänger 50 eines zusätzlichen mobilen Geräts 48 empfangen. Da sich der zusätzliche Signalempfänger 50 nicht an der gleichen Position wie der Signalempfänger 24 befinden kann und zudem wahrscheinlich auch nicht dieselbe Ausrichtung im Raum 14 hat, empfangen der Signalempfänger 24 und der zusätzliche Signalempfänger 50 nicht ein identisches Raumsignal 26.

Im Schritt S32 wird ein zusätzliches Messsignal basierend auf dem von dem zusätzlichen mobilen Gerät 48 empfangenen Raumsignal 26 ausgegeben. Im Schritt S34 wird das Sollwertsignal 38 geändert. Die Änderung des Sollwertsignals 38 kann aber auch zu einem anderen Zeitpunkt durchgeführt werden, wobei die Änderung insbesondere unabhängig davon durchgeführt werden kann, in welchem Verfahrensschritt sich das Verfahren 60, 64 befindet. Der Schritt S34 wurde lediglich für dieses Ausführungsbeispiel nach dem Schritt S32 angeordnet.

Im optionalen Schritt S36 wird aus dem zusätzlichen Messsignal 52 ein zusätzliches Istwertsignal gewonnen. Dieser Schritt wird bevorzugt durchgeführt, wenn das nachfolgende Ansteuern 66 des Zielgeräts 18 dadurch vereinfacht werden kann. In diesem Fall wird dann das Zielgerät 18 in Abhängigkeit davon angesteuert, ob die Änderung des Sollwertsignals 38 zu einer entsprechenden Änderung des zusätzlichen Istwertsignals führt. Die Überprüfung, ob eine entsprechende Änderung erfolgt, erfolgt bevorzugt zeitlich versetzt, um dem Regelsystem etwas Zeit zu geben, die Änderung zu erfassen.

In einem Block 66 wird das Zielgerät 18 oder das zusätzliche Zielgerät 46 durch das zusätzliche mobile Gerät 48 in Abhängigkeit davon angesteuert, ob das Ändern des Sollwertsignals 38 zu einer entsprechenden Änderung des zusätzlichen Messsignals 52 führt bzw. geführt hat. Dabei wird zunächst in einem Vergleichsschritt S38 ermittelt, ob die entsprechende Änderung vorliegt. Ist dies der Fall, verzweigt das Verfahren über den Zweig J zum Schritt S40, in dem es dem zusätzlichen mobilen Gerät 48, insbesondere durch ein Freigabesignal, ermöglicht wird, das Zielgerät 18 oder das zusätzliche Zielgerät 46 zu steuern. Liegt keine entsprechende Änderung vor, so verzweigt das Verfahren über den Zweig N zum Schritt S42, wo es unterbunden wird, dass das zusätzliche mobile Gerät 48 das Zielgerät 18 oder das zusätzliche Zielgerät 46 ansteuert.

Bei dieser Ausgestaltung wird die Präsenz des mobilen Geräts 12 dadurch erkannt, dass das Istwertsignal 36 - welches bei bevorzugten Ausführungsformen dem Messsignal 26 entspricht - dem Sollwertsignal 38 nachgeführt werden kann. Die Präsenz des zusätzlichen mobilen Geräts 48 oder weiterer mobiler Geräte wird dadurch geprüft, ob eine Änderung des Sollwertsignals 38 auch von dem zusätzlichen Signalempfänger 50 des zusätzlichen mobilen Geräts 48 detektiert werden kann.

Fig. 7 zeigt einen ersten beispielhaften Verlauf des Istwertsignals 36. Dabei sind entlang der x-Achse die Zeit t und entlang der y-Achse die Intensität I abgetragen. In einem unteren Teil des Balkens ist jeweils die Grundintensität bzw. das Grundrauschen in dem Raum aufgetragen, das sich z.B. durch einfallendes Tageslicht, bewegliche Leuchten im Raum oder durch Positions- und Ausrichtungsänderungen des Signalempfängers 24 verändern kann. Zum Zeitpunkt t = 1 ist die Signalquelle 20 noch ausgeschaltet, so dass nur die Grundintensität gemessen wird.

Zum Zeitpunkt t = 2 wurde die Signalquelle 20 eingeschaltet, so dass sich zu der Grundintensität ein zusätzlicher Anteil addiert. Wie hier gezeigt ist, führt diese Addition jedoch zu einem deutlichen Überschreiten des Sollwerts 38, so dass in einem nächsten Schritt die Signalquelle 20 für eine geringere Abstrahlung angesteuert wird. Ziel ist es dabei, das Istwertsignal innerhalb des Toleranzfensters 40 einzupegeln.

Nachdem zum Zeitpunkt t = 3 der Istwert 36 unterhalb des Toleranzfensters 40 liegt und zum Zeitpunkt t = 4 oberhalb des Toleranzfensters 40 liegt, liegt das Istwertsignal 36 zu den Zeitpunkten t = 5, t = 6 und t = 7 innerhalb des Toleranzfensters 40. Die Annäherung des Istwertsignals 36 an den Sollwert 38 hat also innerhalb des Abstands D und innerhalb der maximalen Zeit T stattgefunden. In der Figur ist der Abstand D zweimal, um einer Abweichung in positiver Richtung um den Abstand D und einer Abweichung in negativer Richtung um den Abstand D Rechnung zu tragen. Optional kann nachfolgend in einem weiteren Toleranzfenster 70 überprüft werden, ob die Annäherung bestehen bleibt.

Fig. 8 zeigt ein zweites Beispiel eines Verlaufs des Istwertsignals 36. Es ist zu erkennen, dass das Istwertsignal 36 nicht dem Sollwertsignal 38 angenähert werden kann. Dies erlaubt den Rückschluss, dass sich das mobile Gerät 12 nicht in dem Raum 14 befindet.

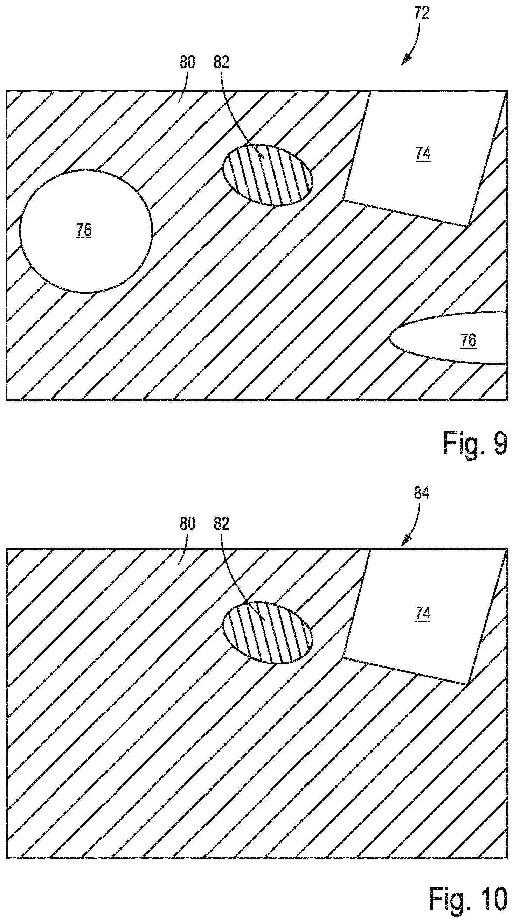

Fig. 9 zeigt ein Ausführungsbeispiel eines Bilds 72 aus einer zeitlichen Abfolge von Bildern. Die Abfolge von Bildern wird mit einem als Kamera ausgebildeten Signalempfänger 24 gewonnen und im Messsignal 32 übertragen. Das Bild 72 zeigt hier einen ersten Bereich 74, einen zweiten Bereich 76 und einen dritten Bereich 78, die jeweils eine hohe Intensität anzeigen. Ferner gibt es einen großen vierten Bereich 80 mit einer mittleren Intensität und einen fünften Bereich 82 mit geringer Intensität. Das Bild 72 wurde in einem Raum 14 eingenommen, in dem eine gepulste Signalquelle 20, also eine pulsweitenmodulierte Signalquelle 20, verwendet wird.

Fig. 10 zeigt ein zweites Bild 84 aus einer beispielhaften zeitlichen Abfolge von Bildern. Im Vergleich zu dem in Fig. 9 gezeigten Bild fehlen hier der zweite Bereich 74 und der dritte Bereich 76. Da die einzelnen Bilder mit einem geringen zeitlichen Abstand von der Kamera aufgezeichnet werden, deuten die Änderungen zwischen dem Bild 72 aus Fig. 9 und dem Bild 84 aus Fig. 10 auf eine schnell stattfindende Änderung hin. Bei bevorzugten Ausgestaltungen werden die einzelnen Bilder mit einem zeitlichen Abstand von weniger als 500 ms, bevorzugt weniger als 200 ms, besonders bevorzugt weniger als 100 ms und insbesondere von weniger als 50 ms gewonnen.

Aufgrund einer Analyse der Unterschiede zwischen den Bildern 72, 84 aus Fig. 9 und Fig. 10 kann man zurückschließen, dass der zweite Bereich 76 und der dritte Bereich 78 durch das Pilotsignal 22 hervorgerufen werden. Bei einer Auswertung des Bilds gemäß Fig. 9 kann man die anderen stationären Bildbereiche - den ersten Bereich 74, den vierten Bereich 80 und den fünften Bereich 82 - subtrahieren. Man erhält aufgrund der Differenzanalyse also lediglich die dynamisch veränderlichen Bereiche 76, 78 und kann sich bevorzugt bei der Ermittlung des Istwertsignals 36 auf die vom Pilotsignal 22 besonders erhellten Bereiche des Bildes konzentrieren.

Bei einer bevorzugten Ausgestaltung werden mehrere Bilder auf diese Weise analysiert, um die von der Signalquelle 20 verursachte Intensität ermitteln zu können. Je mehr Bilder die dynamischen Bereiche 76, 78 aufzeigen in Relation zu der Gesamtzahl der analysierten Bilder, desto größer ist die von der Signalquelle 20 hervorgerufene Intensität. Auf diese Weise kann das Istwertsignal 36 bei bevorzugten Ausgestaltungen ermittelt werden, um im Hinblick auf das zu erreichende Sollwertsignal 38 die Ansteuerung der Signalquelle 20 korrigieren zu können.

## Patentansprüche

1. Vorrichtung (10) zur Präsenzerkennung von einem mobilen Gerät (12) in einem Raum (14), die Vorrichtung mit
- einer Gerätesteuereinheit (16) zum Ansteuern eines Zielgeräts (18),
- einem mobilen Gerät (12) zum Ansteuern des Zielgeräts (18) über die Gerätesteuereinheit (16),
- einer Signalquelle (20) zum Aussenden eines Pilotsignals (22),
- einem Signalempfänger (24) zum Empfangen eines durch das Pilotsignal (22) hervorgerufenen Raumsignals (26), wobei der Signalempfänger (24) derart in räumlicher Nähe zu dem mobilen Gerät (12) angeordnet ist, dass sich der Signalempfänger (24) und das mobile Gerät (12) im normalen Arbeitseinsatz gemeinsam in dem Raum befinden, und dafür ausgebildet ist, ein Messsignal (32) in Reaktion auf das empfangene Raumsignal (26) auszugeben,
- einer Signalsteuereinheit (34) zum Ansteuern der Signalquelle,
**gekennzeichnet dadurch, dass**
- die Signalsteuereinheit (34) dafür ausgebildet ist, die Signalquelle (20) in Abhängigkeit von einem aus dem Messsignal (32) gewonnenen Istwertsignal (36) so anzusteuern, dass über der Zeit eine Annäherung des Istwertsignals an ein vorgebbares Sollwertsignal stattfindet, und
- die Gerätesteuereinheit dafür ausgebildet ist, eine Ansteuerung des Zielgeräts durch das mobile Gerät in Abhängigkeit davon vorzunehmen, ob die Annäherung stattgefunden hat, welche ein Indiz dafür ist, dass sich das mobile Gerät (12) in demselben Raum befindet wie die Signalquelle (20)
- wobei die Signalquelle (20) dafür ausgebildet ist, ein optisches Signal auszusenden und der Signalempfänger (24) dafür ausgebildet ist, ein optisches Signal zu empfangen.

2. Vorrichtung nach Anspruch 1, wobei das optische Signal ein Infrarotsignal ist.

3. Vorrichtung nach einem der vorhergehenden Ansprüche, wobei das Istwertsignal (36) ein Infrarotanteil des detektierten Raumsignals (26) ist.

4. Vorrichtung nach einem der vorhergehenden Ansprüche, wobei das Istwertsignal (36) ein Istwert der Luminanz oder ein Istwert der Helligkeit oder ein Istwert der Intensität des detektierten Raumsignals (26) ist.

5. Vorrichtung nach einem der vorhergehenden Ansprüche, wobei die Gerätesteuereinheit (16) dafür ausgebildet ist, zu ermitteln, ob die Annäherung innerhalb eines Toleranzfensters (40) erfolgt, wobei das Toleranzfenster (40) durch eine vorgebbare Höchstdauer (T) für die Annäherung und/oder einen vorgebbaren Höchstabstand (D) von Istwertsignal (36) und Sollwertsignal (38) definiert ist.

6. Vorrichtung nach einem der vorhergehenden Ansprüche, wobei die Vorrichtung (10) dafür ausgebildet ist, das Sollwertsignal (38) über der Zeit (t) zu ändern.

7. Vorrichtung nach einem der vorhergehenden Ansprüche, wobei das Messsignal (32) eine zeitliche Abfolge des vom Signalempfänger (24) empfangenen Raumsignals (26) repräsentiert.

8. Vorrichtung nach einem der vorhergehenden Ansprüche, wobei der Signalempfänger (24) eine Kamera ist und, insbesondere, das Messsignal (32) eine zeitliche Abfolge von Bildern ist, die von der Kamera aufgenommen wurden.

9. Vorrichtung nach einem der vorhergehenden Ansprüche, wobei die Signalquelle (20) dafür ausgebildet ist, eine gewünschte Signalstärke durch Abgabe einer variablen Sendeleistung und/oder durch eine entsprechend gepulste konstante Sendeleistung bereitzustellen.

10. Vorrichtung nach einem der vorhergehenden Ansprüche, wobei der Signalempfänger eine im mobilen Gerät (12) eingebaute Kamera ist, der ein infrarotdurchlassendes Filter (28) aufgesetzt ist, und wobei, insbesondere, zusätzlich eine Weitwinkellinse (30) aufgesetzt ist.

11. Vorrichtung nach einem der vorhergehenden Ansprüche, wobei die räumliche Nähe derart ausgestaltet ist, dass sich das mobile Gerät (12) und der Signalempfänger (24) in einem Abstand von weniger als 5 m, bevorzugt weniger als 2 m, besonders bevorzugt weniger als 1 m und insbesondere weniger als 0,5 m zueinander befinden.

12. Vorrichtung nach einem der vorhergehenden Ansprüche, wobei die räumliche Nähe derart ausgestaltet ist, dass der Signalempfänger (24) in das mobile Gerät (12) eingebaut ist.

13. Vorrichtung nach einem der vorhergehenden Ansprüche, wobei die Vorrichtung (10) ein zusätzliches mobiles Gerät (48) mit einem zusätzlichen Signalempfänger (50) zum Empfangen des Raumsignals (26) aufweist, wobei der zusätzliche Signalempfänger (50) dafür ausgebildet ist, ein zusätzliches Messsignal (52) in Reaktion auf das empfangene Raumsignal (26) auszugeben, und wobei die Gerätesteuereinheit (16) ferner dafür ausgebildet ist, eine Ansteuerung des Zielgeräts (18) oder eines zusätzlichen Zielgeräts (46) durch das zusätzliche mobile Gerät (48) in Abhängigkeit davon vorzunehmen, ob eine Änderung des Sollwertsignals (38) zu einer entsprechenden Änderung des zusätzlichen Messsignals (52) führt.

14. Verfahren (60) zur Präsenzerkennung von einem mobilen Gerät (12) in einem Raum (14), das Verfahren mit den Schritten:
- Ansteuern (S10) einer Signalquelle (20) zum Aussenden eines Pilotsignals (22),
- Empfangen (S12), durch einen Signalempfänger (24), eines durch das Pilotsignal (22) hervorgerufenen Raumsignals (26), wobei der Signalempfänger (24) derart in räumlicher Nähe zu dem mobilen Gerät (12) angeordnet ist, dass sich der Signalempfänger (24) und das mobile Gerät (12) im normalen Arbeitseinsatz gemeinsam in dem Raum befinden
- Ausgeben (S14) eines Messsignals (32) basierend auf dem von dem mobilen Gerät (12) empfangenen Raumsignal (26),
- Gewinnen (S16) eines Istwertsignals (36) aus dem Messsignal (26),
- Vergleichen (S18) des Istwertsignals (36) mit einem vorgebbaren Sollwertsignal (38),
**gekennzeichnet durch**
- Anpassen (S20) des Ansteuerns der Signalquelle (20), so dass über der Zeit eine Annäherung des Istwertsignals (36) an das vorgebbare Sollwertsignal (38) stattfindet, und
- Ansteuern (62) des Zielgeräts (18) durch das mobile Gerät (12) in Abhängigkeit davon, ob die Annäherung stattgefunden hat, welche ein Indiz dafür ist, dass sich das mobile Gerät (12) in demselben Raum befindet wie die Signalquelle (20),
- wobei die Signalquelle (20) ein optisches Signal aussendet und der Signalempfänger (24) ein optisches Signal empfängt.

15. Verfahren (64) nach Anspruch 14, ferner mit den Schritten:
- Ausgeben (S32) eines zusätzlichen Messsignals (52) basierend auf dem von einem zusätzlichen mobilen Gerät (48) empfangenen Raumsignal (26),
- Ändern (S34) des Sollwertsignals (38),
- Ansteuern (66) des Zielgeräts (18) oder eines zusätzlichen Zielgeräts (46) durch das zusätzliche mobile Gerät (48) in Abhängigkeit davon, ob das Ändern des Sollwertsignals (38) zu einer entsprechenden Änderung des zusätzlichen Messsignals (52) führt.

## Claims

1. Apparatus (10) for detecting the presence of a mobile device (12) in a room (14), the device having
- an device control unit (16) for controlling a target device (18),
- a mobile device (12) for controlling the target device (18) via the device control unit (16),
- a signal source (20) for emitting a pilot signal (22),
- a signal receiver (24) for receiving a room signal (26) caused by the pilot signal (22), the signal receiver (24) being arranged in spatial proximity to the mobile device (12) such that the signal receiver (24) and the mobile device (12) are together in the room during normal operation, and being adapted to output a measurement signal (32) in response to the received room signal (26),
- a signal control unit (34) for controlling the signal source,
**characterized in that**
- the signal control unit (34) is configured to control the signal source (20) as a function of an actual value signal (36) obtained from the measurement signal (32) in such a way that an approximation of the actual value signal to a predeterminable desired value signal takes place over time, and
- the device control unit is adapted to make the target device be controlled by the mobile device depending on whether the approximation has taken place, which is an indication that the mobile device (12) is in the same room as the signal source (20)
- wherein the signal source (20) is adapted to transmit an optical signal and the signal receiver (24) is adapted to receive an optical signal.

2. Apparatus as claimed in claim 1, where the optical signal is an infrared signal.

3. Apparatus according to one of the preceding claims, wherein the actual value signal (36) is an infrared portion of the detected room signal (26).

4. Apparatus according to one of the preceding claims, wherein the actual value signal (36) is an actual value of luminance or an actual value of brightness or an actual value of the intensity of the detected room signal (26).

5. Apparatus according to one of the preceding claims, the device control unit (16) being designed to determine whether the approximation takes place within a tolerance window (40), the tolerance window (40) being defined by a predeterminable maximum duration (T) for the approximation and/or a predeterminable maximum distance (D) between the actual value signal (36) and the desired value signal (38).

6. Apparatus according to one of the preceding claims, the apparatus (10) being adapted to change the desired signal (38) overtime (t).

7. Apparatus according to one of the preceding claims, wherein the measurement signal (32) represents a temporal sequence of the room signal (26) received by the signal receiver (24).

8. Apparatus according to one of the preceding claims, wherein the signal receiver (24) is a camera and, in particular, the measurement signal (32) is a temporal sequence of images taken by the camera.

9. Apparatus according to one of the preceding claims, wherein the signal source (20) is adapted to provide a desired signal strength by outputting a variable transmission power and/or by a correspondingly pulsed constant transmission power.

10. Apparatus according to one of the preceding claims, wherein the signal receiver is a camera which is built into the mobile device (12) and which is fitted with an infrared-transmitting filter (28), and wherein, in particular, a wide-angle lens (30) is additionally fitted to it.

11. Apparatus according to one of the preceding claims, the spatial proximity being designed in such a way that the mobile device (12) and the signal receiver (24) are located at a distance of less than 5 m, preferably less than 2 m, particularly preferably less than 1 m and in particular less than 0.5 m from one another.

12. Apparatus according to one of the preceding claims, wherein the spatial proximity is configured such that the signal receiver (24) is built into the mobile device (12).

13. Apparatus according to one of the preceding claims, the apparatus (10) comprising an additional mobile device (48) having an additional signal receiver (50) for receiving the room signal (26), the additional signal receiver (50) being adapted to output an additional measurement signal (52) in response to the received room signal (26), and wherein the device control unit (16) is further adapted to control the target device (18) or an additional target device (46) by the additional mobile device (48) depending on whether a change in the desired signal (38) results in a corresponding change in the additional measurement signal (52).

14. Method (60) for detecting the presence of a mobile device (12) in a room (14), the method comprising the steps of:
- controlling (S10) a signal source (20) to emit a pilot signal (22),
- receiving (S12), by a signal receiver (24), a room signal (26) caused by the pilot signal (22), the signal receiver (24) being arranged in spatial proximity to the mobile device (12) in such a way that the signal receiver (24) and the mobile device (12) are located together in the room during normal working operation,
- outputting (S14) a measurement signal (32) based on the room signal (26) received from the mobile device (12),
- obtaining (S16) an actual value signal (36) from the measuring signal (26),
- comparing (S18) the actual value signal (36) with a predeterminable desired signal (38),
**characterized by**
- adapting (S20) the control of the signal source (20) so that an approximation of the actual value signal (36) to the predeterminable desired signal (38) takes place over time, and
- controlling (62) the target device (18) by the mobile device (12) depending on whether the approximation has taken place, which is an indication that the mobile device (12) is in the same room as the signal source (20),
- wherein the signal source (20) emits an optical signal and the signal receiver (24) receives an optical signal.

15. Method (64) according to claim 14, further with the steps:
- outputting (S32) an additional measurement signal (52) based on the room signal (26) received from an additional mobile device (48),
- changing (S34) the desired signal (38),
- controlling (66) the target device (18) or an additional target device (46) by the additional mobile device (48) depending on whether changing the desired signal (38) results in a corresponding change of the additional measurement signal (52).

## Revendications

1. Dispositif (10) pour détecter la présence d'un dispositif mobile (12) dans un local (14), le dispositif comportant
- une unité de commande de dispositif (16) pour commander un dispositif cible (18),
- un dispositif mobile (12) pour commander le dispositif cible (18) via l'unité de commande de dispositif (16),
- une source de signal (20) pour émettre un signal pilote (22),
- un récepteur de signaux (24) pour recevoir un signal d'ambiance (26) provoqué par le signal pilote (22), le récepteur de signaux (24) étant disposé à proximité spatiale du dispositif mobile (12) de telle sorte que le récepteur de signaux (24) et le dispositif mobile (12) sont ensemble dans l'espace en fonctionnement normal, et étant adapté pour émettre un signal de mesure (32) en réponse au signal d'ambiance reçu (26),
- une unité de commande de signal (34) pour commander la source de signal,
**caractérisé en ce que**
- l'unité de commande de signal (34) est conçue pour commander la source de signal (20) en fonction d'un signal de valeur réelle (36) obtenu à partir du signal de mesure (32) de telle sorte qu'une approximation du signal de valeur réelle à un signal de valeur prescrite prédéterminé s'effectue dans le temps, et
- l'unité de commande de dispositif est adaptée pour que le dispositif cible soit commandé par le dispositif mobile selon que l'approximation a eu lieu, ce qui est une indication que le dispositif mobile (12) est dans le même espace que la source de signal (20)
- dans laquelle la source de signal (20) est adaptée pour transmettre un signal optique et le récepteur de signal (24) est adapté pour recevoir un signal optique.

2. Dispositif selon la revendication 1, dans lequel le signal optique est un signal infrarouge.

3. Dispositif selon l'une des revendications précédentes, dans lequel le signal de valeur réelle (36) est une partie infrarouge du signal spatial détecté (26).

4. Dispositif selon l'une des revendications précédentes, dans lequel le signal de valeur réelle (36) est une valeur réelle de luminance ou une valeur réelle de luminosité ou une valeur réelle d'intensité du signal de pièce détecté (26).

5. Dispositif selon l'une des revendications précédentes, l'unité de commande de dispositif (16) étant conçue pour déterminer si l'approximation a lieu dans une fenêtre de tolérance (40), la fenêtre de tolérance (40) étant définie par une durée maximale prédéterminée (T) pour l'approximation et/ou une distance maximale prédéterminée (D) entre le signal de valeur réelle (36) et le signal de valeur prescrite (38).

6. Dispositif selon l'une des revendications précédentes, le dispositif (10) étant adapté pour modifier le signal de consigne (38) dans le temps (t).

7. Dispositif selon l'une des revendications précédentes, dans lequel le signal de mesure (32) représente une séquence temporelle du signal spatial (26) reçu par le récepteur de signal (24).

8. Dispositif selon l'une des revendications précédentes, dans lequel le récepteur de signaux (24) est une caméra et en particulier le signal de mesure (32) est une suite temporelle d'images prises par la caméra.

9. Dispositif selon l'une des revendications précédentes, dans lequel la source de signal (20) est adaptée pour fournir une intensité de signal souhaitée en délivrant une puissance de transmission variable et/ou une puissance de transmission constante pulsée correspondante.

10. Dispositif selon l'une des revendications précédentes, dans lequel le récepteur de signaux est une caméra qui est intégrée dans le dispositif mobile (12) et qui est équipée d'un filtre (28) de transmission infrarouge, et en ce que, en particulier, un objectif grand angle (30) est également monté.

11. Dispositif selon l'une des revendications précédentes, la proximité spatiale étant conçue de telle sorte que le dispositif mobile (12) et le récepteur de signaux (24) soient situés à une distance inférieure à 5 m, de préférence inférieure à 2 m, de plus préférence inférieure à 1 m et en particulier inférieure à 0,5 m.

12. Dispositif selon l'une des revendications précédentes, dans lequel la proximité spatiale est conçue de telle sorte que le récepteur de signal (24) est intégré dans le dispositif mobile (12).

13. Dispositif selon l'une des revendications précédentes, le dispositif (10) comprenant un dispositif mobile supplémentaire (48) ayant un récepteur de signal supplémentaire (50) pour recevoir le signal d'ambiance (26), le récepteur de signal supplémentaire (50) étant adapté pour délivrer un signal de mesure supplémentaire (52) en réponse au signal d'ambiance (26) reçu, et dans laquelle l'unité de commande de dispositif (16) est en outre adaptée pour commander le dispositif cible (18) ou un dispositif cible supplémentaire (46) par le dispositif mobile supplémentaire (48) selon qu'une modification du signal de valeur prescrite (38) entraîne une modification correspondante du signal de mesure supplémentaire (52).

14. Procédé (60) pour détecter la présence d'un dispositif mobile (12) dans une pièce (14), le procédé comprenant les étapes consistant à :
- commander (S10) une source de signal (20) pour émettre un signal pilote (22),
- recevoir (S12), par un récepteur de signaux (24), un signal d'ambiance (26) provoqué par le signal pilote (22), le récepteur de signaux (24) étant disposé à proximité spatiale du dispositif mobile (12) de telle sorte que le récepteur de signaux (24) et le dispositif mobile (12) se trouvent ensemble dans l'espace en fonctionnement normal,
- sortir (S14) un signal de mesure (32) basé sur le signal spatial (26) reçu du dispositif mobile (12),
- obtenir (S16) un signal de valeur réelle (36) à partir du signal de mesure (26),
- comparer (S18) du signal de valeur réelle (36) avec un signal de valeur prescrite prédéfinissable (38),
**caractérisé par**
- adapter (S20) la commande de la source de signal (20) de sorte qu'une approximation du signal de valeur réelle (36) au signal de valeur prescrite prédéfinissable (38) s'effectue dans le temps, et
- commander (62) du dispositif cible (18) par le dispositif mobile (12) selon que l' approximation a eu lieu, ce qui est une indication que le dispositif mobile (12) est dans le même espace que la source de signal (20),
- dans laquelle la source de signal (20) émet un signal optique et le récepteur de signal (24) reçoit un signal optique.

15. Procédé (64) selon la revendication 14, en plus avec les étapes :
- sortir (S32) un signal de mesure supplémentaire (52) basé sur le signal spatial (26) reçu d'un dispositif mobile supplémentaire (48),
- modifier (S34) le signal de valeur prescrite (38),
- commander (66) le dispositif cible (18) ou d'un dispositif cible supplémentaire (46) par le dispositif mobile supplémentaire (48) selon que la modification du signal de valeur prescrite (38) entraîne une modification correspondante du signal de mesure supplémentaire (52).
